Europäisches Patentamt

European Patent Office

Office européen des brevets

(19)

(11)　EP 0 948 398 B1

(12)　EUROPÄISCHE PATENTSCHRIFT

(45) Veröffentlichungstag und Bekanntmachung des Hinweises auf die Patenterteilung:
　　　**13.10.2004　Patentblatt 2004/42**

(21) Anmeldenummer: **97931787.2**

(22) Anmeldetag: **07.07.1997**

(51) Int Cl.⁷: **B01J 19/00**, B01L 3/00,
　　　C07K 1/04, C07H 21/00,
　　　C03C 17/30

(86) Internationale Anmeldenummer:
　　　**PCT/EP1997/003571**

(87) Internationale Veröffentlichungsnummer:
　　　**WO 1998/003257 (29.01.1998 Gazette 1998/04)**

(54) **FESTE TRÄGER FÜR ANALYTISCHE MESSVERFAHREN, EIN VERFAHREN ZU IHRER HERSTELLUNG SOWIE IHRE VERWENDUNG**

SOLID SUPPORTS FOR ANALYTICAL MEASURING PROCESSES, A PROCESS FOR THEIR PREPARATION, AND THEIR USE

SUPPORTS SOLIDES POUR PROCESSUS DE MESURE ANALYTIQUES, PROCEDE PERMETTANT DE LES OBTENIR ET UTILISATION

(84) Benannte Vertragsstaaten:
　　　**AT BE CH DE ES FI FR GB IT LI NL SE**

(30) Priorität: **18.07.1996　DE 19628928**

(43) Veröffentlichungstag der Anmeldung:
　　　**13.10.1999　Patentblatt 1999/41**

(73) Patentinhaber: **BASF AKTIENGESELLSCHAFT**
　　　**67056 Ludwigshafen (DE)**

(72) Erfinder:
　　　• **EIPEL, Heinz**
　　　　**D-64625 Bensheim (DE)**

• **KELLER, Harald**
　　**D-67069 Ludwigshafen (DE)**

(56) Entgegenhaltungen:
　　　EP-A- 0 402 718　　　WO-A-94/27719
　　　WO-A-95/35505　　　WO-A-97/22875
　　　DE-A- 3 915 920　　　US-A- 4 728 792
　　　US-A- 5 041 266

• **MATSUDA T ET AL: "MICROFABRICATED SURFACE DESIGNS FOR CELL CULTURE AND DIAGNOSIS" ASAIO JOURNAL, Bd. 40, Nr. 3, 1.Juli 1994, Seiten 594-597, XP000498248**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft feste Träger für analytische Meßverfahren, die im wesentlichen aufgebaut sind aus einem inerten festen Trägermaterial, auf dem hydrophile Meßbereiche, die gegebenenfalls mit einer Oberflächenladung versehen sind, durch mindestens eine hydrophobe Beschichtung voneinander getrennt sind, wobei auf dem Träger größer oder gleich 10 Meßpunkte pro cm$^2$ aufgebracht sind. Weiterhin betrifft die Erfindung ein Verfahren zur Herstellung der Träger, sowie die Verwendung der Träger in der Diagnostik, in der Wirkstoffsuchforschung, in der kombinatorischen Chemie, im Pflanzenschutz, in der Toxikologie oder im Umweltschutzbereich.

Eine Hauptaufgabe der Wirkstoffsuchforschung im Pflanzenschutz oder in der Medizin ist die Identifizierung neuer Leitstrukturen und die Entwicklung von wirkstoffen, die aus diesen Strukturen hervorgehen.

In der klassischen Wirkstoffsuchforschung wurde die biologische Wirkung neuer Verbindungen in einem Zufalls-Screening am ganzen Organismus beispielsweise der Pflanze oder dem Mikroorganismus getestet. Dabei wurden komplexe In-vitro- und In-vivo-Testmethoden eingesetzt, mit denen pro Jahr nur einige hundert Substanzen getestet werden konnten.

Die biologische Testung war dabei gegenüber der Synthesechemie der limitierende Faktor.

Durch die Bereitstellung molekularer Testsysteme, die in der Molekular- und Zellbiologie entwickelt wurden, hat sich die Situation drastisch verändert. Diese molekularen Testsysteme wie beispielsweise Rezeptorbindungsassays, Enzymassays oder Zell-Zellinteraktionsassays lassen sich in der Regel gut in Mikrotiterplatten in Reaktionsvolumen zwischen 50 bis 250 ml durchführen und einfach automatisieren. Die Automatisierung und Miniaturisierung dieser Testsysteme ermöglicht einen hohen Probendurchsatz. Durch diese Entwicklung lassen sich große Zahlen verschiedener Chemikalien auf eine mögliche Verwendung als Leitstruktur in der Wirkstoffsuchforschung testen.

Ein modernes automatisiertes Testsystem ermöglicht in einem Massenscreening die Prüfung von 100.000 und mehr Chemikalien pro Jahr auf ihre biologische Wirkung. Mikrotiterplattenassays werden sehr häufig verwendet, da sie in der Regel geringe Kosten verursachen, sehr sicher und wenig störanfällig sind.

Um die Leistungsfähigkeit dieser Testsysteme voll ausschöpfen zu können, wurden und werden immer neue Festphasensynthesen in der kombinatorischen Chemie entwickelt.

Die kombinatorische Chemie ermöglicht die Synthese einer breiten Vielfalt unterschiedlicher chemischer Verbindungen, sogenannter Substanzbibliotheken. Dies gilt insbesondere, wenn sich die kombinatorische Chemie der automatisierten Festphasensynthese bedient (s. z.B. Übersichtsartikel I. Med. Chem. 1994, 37, 1233 und 1994, 37, 1385). Die Synthese an der Festphase hat den Vorteil, daß eine große Vielzahl an Verbindungen synthetisiert werden können und daß Nebenprodukte und überschüssige Reaktanten leicht entfernt werden können, so daß eine aufwendige Reinigung der Produkte nicht notwendig ist.

Durch die Vielzahl der synthetisierten Verbindungen in der kombinatorischen Chemie kann die Leistungsfähigkeit moderner automatisierter Testsysteme von Seite der Chemikalienvielfalt voll ausgenutzt werden. Da aber im Gegensatz zur klassischen Wirkstoffsynthese die zu untersuchenden Chemikalien bei der Synthese mittels kombinatorischer Chemie nicht in beliebiger Menge zur Verfügung stehen, kann aufgrund der erforderlichen Chemikalienmengen in den Testsystemen nur eine eingeschränkte Zahl von Testsystemen geprüft werden.

Ein weiterer Nachteil der vorhandenen Testsysteme beispielsweise in der Wirkstoffsuchforschung, in der Diagnostik, im Umweltschutz oder Pflanzenschutz ist, daß für viele Testsysteme die benötigten Reagentien wie beispielsweise Enzyme, Antikörper, Rezeptoren, Fluoreszenzfarbstoffe, radioaktiv oder sonstig markierte Liganden, Cytokine, Aktivatoren, Inhibitoren oder sonstige Reagentien teuer, schwer herstellbar sind und/oder nicht in ausreichender Menge für die automatisierten Tests zur Verfügung stehen.

In DE-A 44 35 727 wird ein Ansatz zur Reduktion der für einen Test benötigten Reagentien beschrieben.

Nachteil dieses Verfahrens ist, daß der Träger für die Messungen erst aufwendig in einem mehrstufigen Verfahren hergestellt werden muß.

Ein weiterer Nachteil ist, daß die Reaktionen, die mit diesem Trägermaterial durchgeführt werden können, auf festphasengebundene Reaktionen wie die Reaktantenbindung zwischen Antikörpern, Antigenen, Haptenen oder Nucleinsäuren beschränkt sind. Reaktionen in Lösung können mit dieser Methode nicht durchgeführt werden.

In WO 94/27719 wird ein Verfahren zur Durchführung einer großen Zahl von chemischen Reaktionen auf einem festen Träger beschrieben. Im Verfahren werden die Reaktionslösungen auf die funktionalisierten Bindungsstellen des Trägers mit einer piezoelektrischen Pumpe aufgetragen. Dabei besteht der Träger aus hydrophilen Messpunkten, an bzw. in denen die Reaktanten über kovalente oder nicht-kovalente Bindungen fixiert werden und die von einer durchgehenden hydrophoben Beschichtung umgeben sind.

Es bestand daher die Aufgabe, ein neues analytisches Meßverfahren, das ohne die genannten Nachteile durchführbar ist, zu entwickeln und für die Wirkstoffsuchforschung, die Diagnostik, den Umweltschutz, den Pflanzenschutz, der Toxikologie oder für die kombinatorische Chemie zur Verfügung zu stellen.

Die gestellte Aufgabe konnte dadurch gelöst werden, daß man den eingangs beschriebenen festen Träger für das Meßverfahren verwendet.

Es wurde gefunden, daß die Oberflächenspannung, die einer weiteren Miniaturisierung der vorhandenen Mikrotiterplattentechnik zu immer kleineren Reaktionslöchern (= Wells) hin im Wege steht, da durch sie in sehr kleinen Mikrotiterplattenvertiefungen Kräfte wie die Adhäsion der Reaktionsflüssigkeit an die Oberfläche der Mikrotiterplatten oder die Kapillarkräfte eine immer größere Rolle spielen und so eine Befüllung der Reaktionslöcher und damit eine Messung unmöglich machen, für die erfindungsgemäßen Träger vorteilhaft genutzt werden kann.

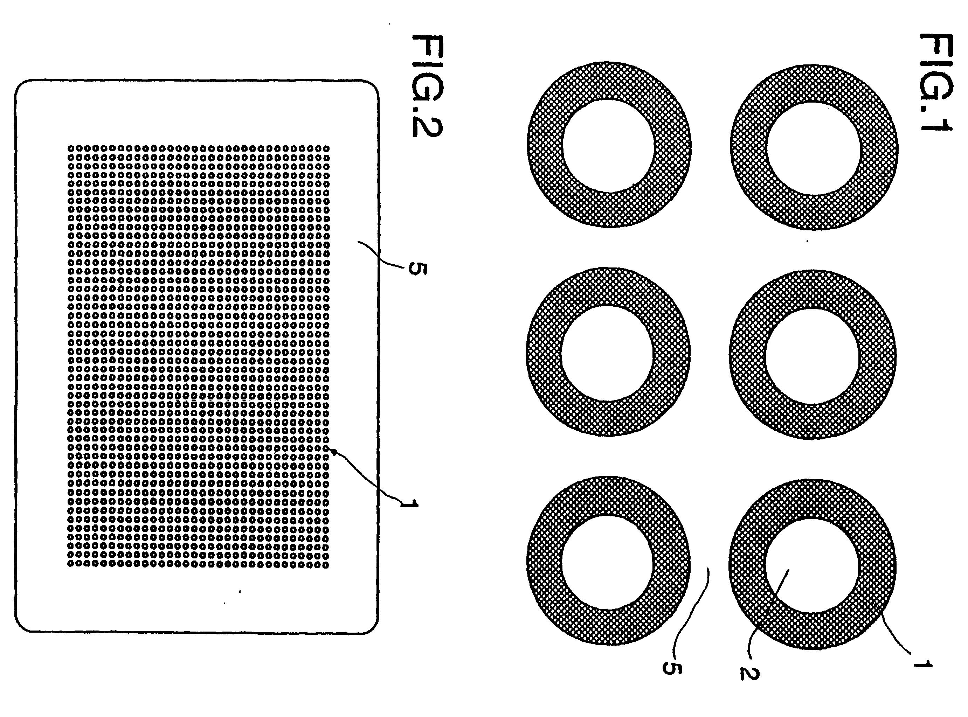

Unter hydrophilen Meßbereichen des Trägers sind Gebiete des Trägers zu verstehen, auf denen bzw. in denen nach Aufbringung der Reaktionsflüssigkeit und damit der Reaktanten die Messung durchgeführt wird (siehe Nummer 2 in den Figuren 1, 3 und 4). Sie entsprechen damit den "Wells" bzw. den Vertiefungen der Mikrotiterplatten und werden nachstehend als "Meßbereiche oder Meßpunkte" bezeichnet.

Die hydrophilen Meßbereiche des Trägers sind vorteilhaft von einem hydrophoben Bereich (siehe Nummer 1 in den Figuren 1 bis 4) umgeben. Dieser hydrophobe Bereich kann aus mindestens einer hydrophoben Beschichtung aufgebaut sein, die den Träger nur teilweise mit Unterbrechungen bedeckt. Diese Unterbrechungen (siehe Nummer 5 in den Figuren 1 bis 4) sind hydrophil.

Die Figuren 1 bis 4 dienen der beispielhaften Verdeutlichung der erfindungsgemäßen Träger.

Die Meßbereiche sowie die sie voneinander trennenden hydrophoben Bereiche (siehe Nummer 1 in den Figuren 1 bis 4) können beispielsweise durch Mikrolithographie-, Photoätz-, Mikrodruck- oder Mikrostempeltechnik aufgebracht werden oder mit Hilfe einer Maskentechnik aufgesprüht werden. Aus der Herstellungstechnik von Druckplatten sind photochemische Verfahren bekannt, mit denen Oberflächen von Platten oder Walzen gezielt an bestimmten Stellen hydrophob und an anderen Stellen hydrophil gemacht werden können. Mit dieser Technik lassen sich beispielsweise auf einfache weise auf einem Träger, z.B. auf einer Glas- oder Metallplatte, ein Raster von mehreren Tausend regelmäßig angeordneten hydrophilen Meßbereichen (siehe Nummer 2 in den Figuren 1, 3 und 4), umgeben von hydrophoben Begrenzungen (siehe Nummer 1 in den Figuren 1 bis 4), herstellen. Dabei können zunächst ein oder mehrere hydrophobe Beschichtungen auf den Träger aufgezogen werden und anschließend die Meßbereiche an den gewünschten Stellen aufgebracht werden oder umgedreht zunächst die hydrophilen Meßbereiche und dann die hydrophoben Bereiche oder beides gleichzeitig aufgezogen werden. Es können auch mehrfach hydrophile Meßbereiche an die gleiche Stelle aufgetragen werden.

Figur 2 gibt beispielhaft einen erfindungsgemäßen Träger in Größe einer Mikrotiterplatte wieder.

Die Meßbereiche können eine beliebige Form haben, bevorzugt sind runde Meßbereiche.

Die hydrophobe Beschichtung oder Beschichtungen können mit beliebig gestalteten Unterbrechungen versehen sein. Sie können auch als separate Bereiche um die Meßbereiche liegen, bevorzugt sind hydrophobe Ringe, die die hydrophilen Meßbereiche voneinander trennen.

Die hydrophobe Beschichtung bzw. Beschichtungen sollen das Verlaufen der Meßbereiche ineinander verhindern und so eine exakte Messung einzelner Reaktionsansätze ermöglichen.

Prinzipiell ist es möglich, jede beliebige Anzahl von Meßpunkten auf einen Träger aufzubringen, bevorzugt sind größer oder gleich 10 Meßpunkte pro $cm^2$, besonders bevorzugt sind größer oder gleich 15 Meßpunkte pro $cm^2$, ganz besonders bevorzugt sind größer oder gleich 20 Meßpunkte pro $cm^2$. Dabei werden Reaktionsvolumina von wenigen nl bis zu einigen ml aufgetragen, bevorzugt werden Volumina kleiner 5 ml, besonders bevorzugt kleiner oder gleich 1 ml, aufgetragen.

Die Meßpunkte können in beliebigen Rastern auf den Träger aufgebracht werden, bevorzugt sind quadratische oder rechteckige Raster.

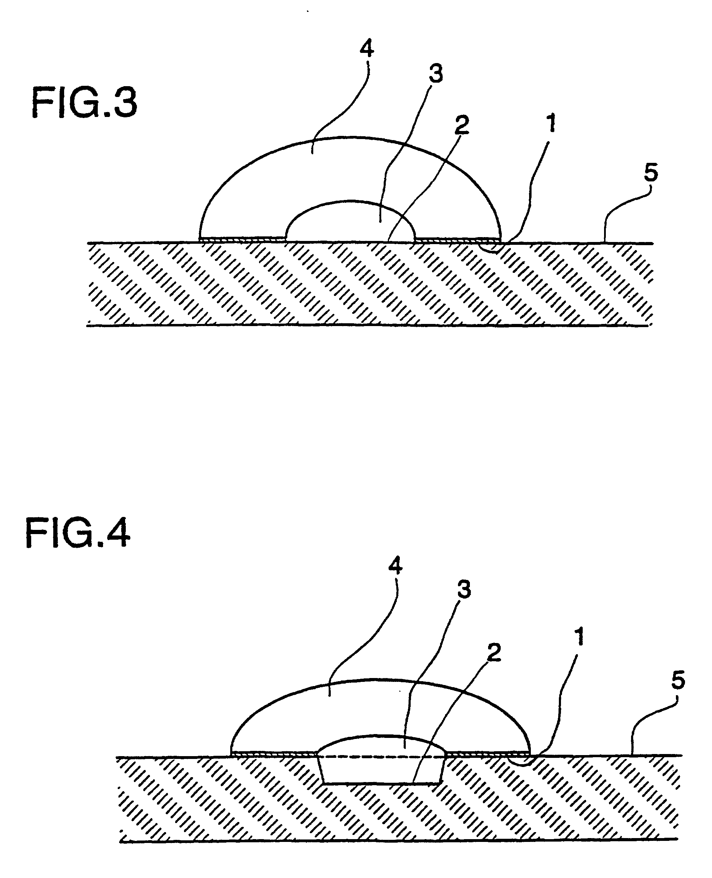

Der inerte feste Träger kann aus einer ebenen, planaren Platte eines eben solchen Blockes oder einer Folie beliebiger Form und Größe bestehen, die gegebenenfalls kleine Mulden (siehe Figur 4) an den Stellen der Meßbereiche haben kann, bevorzugt sind plane Träger (siehe Figur 3). Bevorzugt sind rechteckige oder quadratische Träger, besonders bevorzugt sind rechteckige Träger in Größe einer Standardmikrotiterplatte (127,5 mm x 85,5 mm) oder ganzzahlige Vielfache der Mikrotiterplatten, die größer oder kleiner sein können wie beispielsweise die sogenannten Terasaki-Platten (81 mm x 56 mm, 60 Meßpunkte). Die bevorzugte Größe der erfindungsgemäßen Träger hat den Vorteil, daß die gesamte Peripherie der automatisierten Mikrotiterplattentechnik ohne Umbau verwendet werden kann.

Der Träger kann beispielsweise aus Materialien bestehen wie Glas, Keramik, Quarz, Metall, Stein, Holz, Kunststoff, Gummi, Silicium, Germanium oder Porzellan. Die Materialien können in reiner Form, als Mischungen, Legierungen oder Blends oder in verschiedenen Schichten oder nach Beschichtung beispielsweise mit einem Kunststoff oder einem Lack zur Herstellung der erfindungsgemäßen Träger verwendet werden. Bevorzugt werden transparente Träger aus Quarz, Glas, Kunststoff, Germanium oder Silicium hergestellt, die für alle visuellen Tests wie mikroskopische, kameraunterstützte, laserunterstützte Tests geeignet sind.

Als transparente Kunststoffe sind alle amorphen Kunststoffmaterialien, die einphasig oder mehrphasig mit gleichen Brechungsindex wie Polymere aus Acrylnitril-Butadienstyrol oder mehrphasig mit unterschiedlichem Brechungsindex, bei denen die Domänen der Kunststoffkomponenten Bereiche bilden, die kleiner als die Wellenlänge des Lichts sind wie beispielsweise die Blockcopolymere aus Polystyrol und Butadien (sog. Polystyrol/Butadienblends) geeignet.

Als besonders geeignete transparente Kunststoffe seien hier Polystyrol, Styrolacrylnitril, Polypropylen, Polycarbonat, PVC (= Polyvinylchlorid), Polymethylmethacrylat, Polyester, Silicone, Polyethylenacrylat, Polylactid oder Celluloseacetat, Cellulosepropionat, Cellulosebutyrat oder deren Mischungen genannt.

Silicium- oder Germaniumträger eignen sich besonders für Anwendungen, bei denen eine Detektion oder Induktion der Reaktion über nahes Infrarotlicht erforderlich ist.

Der erfindungsgemäße Träger kann auch in Form eines Transportbandes ausgeführt sein, das bei Automatisierung der Assays an den Beschickungs-, Inkubations- oder Detektionsstationen vorbeilaufen kann.

Die erfindungsgemäßen Träger können beispielsweise ausgehend von Keramik-, Quarz- oder Glasplatten hergestellt werden. Der Träger wird dazu zweckmäßigerweise zunächst mit einem Reinigungsmittel beispielsweise einem Alkohol, einem alkalischen Reiniger oder einem sauren Reiniger wie Reacalc® (enthält laut Angabe der Firma Chemotec GmbH, Phosphorsäure und Tenside) gesäubert. Zur Verbesserung der Reinigung kann diese vorteilhafterweise in einem Ultraschallbad vorgenommen werden. Nach der Reinigung wird der Träger direkt oder nach Spülung mit Wasser und/oder Alkohol oder mit einem Alkohol/Wassergemisch getrocknet. Die hydrophobe Beschichtung des Trägers erfolgt beispielsweise mit einer 1%igen Hexadecyl-trimethoxy-silanlösung in einem Lösungsmittel wie Isopropanol/$H_2O$ (9:1) mit Hilfe einer Stempeltechnik. Der Stempel wird kurz auf den Glasobjektträger zum Aufbringen der 1%igen Hexadecyl-trimethoxy-silanlösung gedrückt. Anschließend wird der Träger getrocknet. Vorteilhafterweise wird der Glasträger bei erhöhten Temperaturen, das heißt bei Temperaturen größer 80 °C, getrocknet. Vorzugsweise wird der Träger nach Trocknung nochmals zur Entfernung von überschüssigem Hexadecyl-trimethoxy-silan gespült beispielsweise mit einer Alkohol/Wassermischung wie Isopropanol/$H_2O$ (9:1).

Gegebenenfalls wird mit dieser Stempeltechnik eine zusätzliche Oberflächenladung im Bereich der hydrophilen Meßpunkte aufgebracht. Diese Oberflächenladung kann beispielsweise durch das Aufbringen von Proteinen, sauren oder basischen Polymeren wie Polylysin oder sauren oder basischen Molekülen erzeugt werden.

Zum Aufbringen von Probenmaterial und Reagenzien eignen sich alle Methoden, die Flüssigkeitsmengen zwischen wenigen nl und wenigen ml dosieren können, wie beispielsweise Techniken, die in Tintenstrahldruckern, sogenannte Ink-jet-Technologie (siehe DE-A 40 24 544) oder in der Durchfluß-Zytometrie, in sogenannten Zellsortern (Heran, P.K., Wheeless, L.L., Quantitative Single Analysis and Sorting, Science 1977, 198, 149 - 157) verwendet werden. Die Tropfenbildung kann dabei mit piezoelektrischer Zertropfung (Ultraschall), piezoelektrischer Tropfenausschleuderung oder Ausschleuderung durch Verdampfung (Ink-Jet-Technik) erfolgen. Es können Systeme mit permanenter Tropfenerzeugung oder Systeme, die Tropfen auf Anforderung erzeugen, verwendet werden.

Mit diesen Techniken können einzelne Tröpfchen exakt dosiert und gezielt auf die einzelnen hydrophilen Meßpunkte der Multi-Analysen-Oberfläche des Trägers plaziert werden, indem zum Beispiel der Träger unter einer oder mehreren parallel angeordneten Düsen entsprechend dem Takt der dosierten Flüssigkeit und entsprechend dem vorgegebenen Raster bewegt wird. Ebenso kann auch die Dosiervorrichtung beispielsweise aus mindestens einer Düse über dem Träger entsprechend dem Takt der dosierten Flüssigkeit und entsprechend dem vorgegebenen Raster bewegt werden.

Es können mit diesen Techniken, falls erforderlich, verschiedene Reagentien und/oder einzelne Zellen an die vorgegebenen Orte (= Meßpunkte) auf der Trägeroberfläche plaziert und zur Reaktion gebracht werden. Von Vorteil ist, daß bei den erfindungsgemäß bevorzugten kleinen Volumina im Bereich von einigen Nanoliter bis zu wenigen Mikroliter eine Vermischung der Reaktanten durch Diffusion sehr rasch eintritt, so daß eine besondere mechanische Mischvorrichtung nicht notwendig ist. Es können auch vor der Zugabe von Flüssigkeitströpfchen zur Durchführung der eigentlichen Analyse gewisse Liganden, z. B. Proteine oder Nucleinsäuren, auf dem Träger in adsorbierter oder chemisch gebundener Form vor der Zudosierung der Meßproben und der Reagentien bereits vorliegen.

Weitere Vorteile der erfindungsgemäßen Träger sind die Einsparung an Substanzen wie beispielsweise an zu testenden Chemikalien, an Enzymen, an Zellen oder sonstigen Reaktanten, an Zeit durch eine weitere Steigerung gegebenenfalls automatisierter paralleler Reaktionsansätze, an Platz- und Personalbedarf, durch eine weitere Miniaturisierung der Reaktionsansätze und dadurch letztlich auch an Geld.

Die auf den Trägern abgelegten Tröpfchen können auch in Form von Geltröpfchen auf den Träger aufgebracht werden, die sich gegebenenfalls anschließend verfestigen und so die Verdunstung der Reaktionsflüssigkeit verringern.

Die Verdunstung der Reaktionsflüssigkeit (siehe Nummer 3 in den Figuren 3 und 4) kann auch durch Überschichtung mit einer hydrophoben Flüssigkeit (siehe Nummer 4 in den Figuren 3 und 4), wobei die hydrophobe Beschichtung oder Beschichtungen wie ein Anker wirken, verringert werden (Figur 3 und 4). Bevorzugt zur Überschichtung werden niedrigviskose Öle wie Silikonöle verwendet.

Die Verdunstung kann auch durch Inkubation der Träger in einer nahezu wasserdampfgesättigten Atmosphäre reduziert werden.

Durch Kühlung der Träger kann die Verdunstung ebenfalls reduziert werden.

Es können einzelne der genannten Elemente zur Verminderung der Verdunstung verwendet werden oder deren Kombinationen.

Die erfindungsgemäßen Träger eignen sich prinzipiell für alle heute in Mikrotiterplatten durchgeführten Analysenmethoden, wie beispielsweise kolorimetrische, fluorimetrische oder densitometrische Methoden. Dabei können die Lichtstreuung, Trübung, wellenlängenabhängige Lichtabsorption, Fluoreszenz, Lumineszenz, Raman-Streuung, ATR (= Attenuated Total Reflection), Radioaktivität, Isotopenmarkierung, pH-Veränderungen oder Ionenverschiebungen vorteilhaft alleine oder in Kombination genutzt und gemessen werden, um hier nur einige der möglichen Meßgrößen zu nennen.

Als mögliche auf den erfindungsgemäßen Trägern durchführbare Analysenmethoden seien hier die Bindung von Antikörper an Antigene, die Wechselwirkung zwischen Rezeptoren und Liganden, die spezifische Spaltung von Substratmolekülen durch Enzyme, die Polymerase-Kettenreaktion ("PCR"), Agglutinationstests oder die Wechselwirkung zwischen verschiedenen oder gleichen Zelltypen wie Enzymassays, Titrationsassays wie Virustitrationsassays, Erythrozyten- oder Plättchenaggregationsassays, Agglutinationsassays mit Latexkügelchen, ELISA- (= Enzyme-linked immunosorbent assay) oder RIA- (= Radioimmunoassay) genannt.

Die erfindungsgemäßen Träger können beispielsweise in der Diagnostik, in der Wirkstoffsuchforschung, in der kombinatorischen Chemie, im Pflanzenschutz, in der Toxikologie, im Umweltschutz beispielsweise bei cytotoxikologischen Tests, in der Medizin oder der Biochemie eingesetzt werden.

Die erfindungsgemäßen Träger sind besonders für das Massenscreening geeignet.

Besonders geeignet sind die erfindungsgemäßen Träger für alle modernen bilderfassenden und bildauswertenden Analysensysteme.

Die folgenden Beispiele dienen der weiteren Veranschaulichung der Erfindung, ohne sie in irgendeiner Weise einzuschränken.

Beispiel 1

Herstellung eines erfindungsgemäßen Trägers aus einem Glasobjektträger

Zunächst wurde der Glasobjektträger mit einer 20%igen wässrigen Lösung eines sauren Reinigers (Reacalc®, Firma Chemotec GmbH) in einem Ultraschalltauchbad 10 Minuten gereinigt. Anschließend wurde der Glasobjektträger mit Wasser und danach mit absolutem Ethanol gespült und bei ca. 23 °C getrocknet.

Mit einem Mikrostempel wurde auf dem hydrophilen Träger die hydrophobe Beschichtung in Form von hydrophoben Ringen (siehe Figur 1 bis 4) aufgebracht. Zur Aufbringung der hydrophoben Schicht wurde eine 1%ige Hexadecyl-trimethoxy-silanlösung in Isopropanol/$H_2O$ (9:1) verwendet. Der in die Silanlösung getauchte Stempel wurde kurz - ca. 5 sec. - auf den Träger gedrückt und anschließend wurde der Träger 15 Minuten bei 100 °C getrocknet. Überschüssige Silanlösung wurde durch Eintauchen des Trägers für ca. 1 Minute in Isopropanol/$H_2O$ (9:1) vom Träger entfernt. Es wurden zwei Typen von Stempeln zum Aufbringen von 12 bzw. 25 Meßpunkten pro Quadratzentimeter verwendet.

Beispiel 2

Protease-Inhibitor-Assay mit den erfindungsgemäßen Trägern

Mit einem nach dem in Beispiel 1 beschriebenen Verfahren hergestellten Träger wurde ein Protease-Inhibitor-Assay durchgeführt.

In einer Kammer mit größer 95 % relativer Luftfeuchtigkeit wurden mit einem Mikro-Dosiersystem von der Firma Microdrop, Norderstedt, auf einem wie oben beschrieben hergestellten Objektträger 96 Proben mit jeweils 100 nl einer Lösung von Fluorescein-isothiocyanat-markiertem Casein (20 µg/ml) in 10 mM Tris/HCl-Puffer (pH 8,5) aufgebracht. Die Anordnung der Reaktionströpfchen erfolgte entsprechend der durch den Stempel aufgebrachten, hydrophoben Bereiche (= Sperrschichten) in 8 Reihen und 12 Spalten in einem Raster von 2 x 2 mm. Die Breite der hydrophoben Ringe war jeweils 0,4 mm.

Anschließend wurde je 1 nl verschiedener Protease-Inhibitoren in einer 1 mM Konzentration in 10 mM Tris/HCl-Puffer (pH 8,5) mit dem Microdrop-Gerät zu den Reaktionsproben zugegeben. Die Zugabe erfolgte exakt in die vorher aufgebrachte fluoreszenzmarkierte Caseinlösung. Als Kontrolle wurde ein Nanoliter 10 mM Tris/HCl-Puffer (pH 8,5) verwendet.

Zum Schluß wurden 10 nl der Protease Trypsin in einer Konzentration von 10 mg/ml in Tris/HCl-Puffer (pH 8,5) zur Reaktion gegeben.

Die Reaktionströpfchen wurden anschließend zur Reduktion der Verdunstung entweder mit Mineralöl, Silikonöl oder mit Paraffin-Öl, das mit dem Microdrop-Dosiersystem aufgebracht wurde, abgedeckt.

Nach 30 Minuten Inkubationszeit wurde der Assay gemessen. Hierzu wurde von der Objektträgerunterseite mit Hilfe eines Invert-Fluoreszenzmikroskops mit linear polarisiertem Licht im Bereich von 450 bis 485 nm Fluoreszenz angeregt und im Bereich von 515 bis 530 nm detektiert. Zur Detektion diente eine kühlbare CCD-Kamera mit vorgeschaltetem motorisch drehbarem Polarisationsfilter.

Es wurde die Anisotropie der Polarisation der Caseinmoleküle nach folgenden Gleichungen bestimmt:

$$A = \frac{I_{senkrecht} - I_{parallel}}{I_{senkrecht} + 2 \times I_{parallel}} \qquad (I)$$

$$P = \frac{I_{senkrecht} - I_{parallel}}{I_{senkrecht} + I_{parallel}} \qquad (II)$$

hierbei bedeutet:

A      die Anisotropie

P      die Polarisation

$I_{parallel}$      die gemessene Intensität des Fluoreszenz-Lichtes bei einer Polarisation parallel zur Polarisation des Anregungslichtes und

$I_{senkrecht}$      die gemessene Intensität des Fluoreszenz-Lichtes bei gekreuzten Polarisationsfiltern

Die Anisotropie ist ein Maß für die rotatorische Diffusionskonstante von Molekülen und kann als Maß zur Abschätzung der hydrodynamischen Molekülgrößen eingesetzt werden (G. Weber, Biochemie, Vol. 51, 1952, 145 - 155).

Wurde das Fluorescein-isothiocyanat-markierte Protein von der Protease gespalten, so wurde eine Polarisation im Bereich von 50 bis 75 x $10^{-3}$ gemessen. Wurde die Protease Trypsin inhibiert, so wurde eine Polarisation größer 150 x $10^{-3}$ gemessen.

Zur parallelen Auswertung aller 96 Reaktionsansätze wurde mit einem Objektiv aus einer Stereo-Lupe das gesamte Meßfeld mit den 96 Meßpunkten gleichzeitig erfaßt und mit einem Bildverarbeitungsprogramm ausgewertet.

Beispiel 3

Protease-Inhibitor-Assay mit 1536 parallelen Meßpunkten

Es wurde ein Trypsin-Inhibitorassay wie unter Beispiel 2 beschrieben mit 1536 parallelen Meßpunkten auf der Größe einer Mikrotiterplatte (siehe Figur 2) durchgeführt.

**Patentansprüche**

1. Fester Träger für analytische Meßverfahren, der im wesentlichen aufgebaut ist aus einem inerten festen Trägermaterial, auf dem hydrophile Meßbereiche, die gegebenenfalls mit einer Oberflächenladung versehen sind, durch mindestens eine unterbrochene hydrophobe Beschichtung voneinander getrennt sind, wobei auf dem Träger größer oder gleich 10 Meßpunkte pro $cm^2$ aufgebracht sind.

2. Fester Träger nach Anspruch 1, **dadurch gekennzeichnet, daß** die auf dem Träger aufgebrachten hydrophilen Meßbereiche durch unterbrochene hydrophobe Bereiche in Form von Ringen voneinander getrennt sind.

3. Träger nach Anspruch 1 oder 2, **dadurch gekennzeichnet, daß** man als Trägermaterial Glas, Keramik, Quarz, Metall, Stein, Kunststoff, Gummi, Silicium oder Porzellan verwendet.

4. Träger nach den Ansprüchen 1 bis 3, **dadurch gekennzeichnet, daß** man ein transparentes Trägermaterial ausgewählt aus der Gruppe Glas, Quarz, Silicium oder Kunststoff verwendet.

5. Analytisches Meßverfahren, **dadurch gekennzeichnet, daß** man auf einem Träger gemäß den Ansprüchen 1 bis 4 flüssige Analysenproben in den hydrophilen Meßbereichen aufbringt, die hydrophilen Messbereiche mit einer hydrophoben Flüssigkeit überschichtet und analysiert.

6. Analytisches Meßverfahren nach Anspruch 5, **dadurch gekennzeichnet, daß** man die analytische Messung in nahezu wasserdampfgesättigter Atmosphäre durchführt.

7. Analytisches Meßverfahren nach Anspruch 5 oder 6, **dadurch gekennzeichnet, daß** man die analytische Messung unter Kühlung des Trägers durchführt.

8. Verwendung eines Trägers gemäß den Ansprüchen 1 bis 4 in der Diagnostik, in der Wirkstoffsuchforschung, in der kombinatorischen Chemie, im Pflanzenschutz, in der Toxikologie oder im Umweltschutzbereich.

**Claims**

1. A solid support for analytical measurement methods which is essentially composed of an inert solid support material on which hydrophilic measurement zones which may be provided with a surface loading are separated from one another by at least one non-continuous hydrophobic coating, where the number of measurement points applied per $cm^2$ of the support is greater than or equal to 10.

2. A solid support as claimed in claim 1, wherein the hydrophilic measurement zones applied to the support are separated from one another by non-continuous hydrophobic zones in the form of rings.

3. A support as claimed in claim 1 or 2, wherein the support material used is glass, ceramic, quartz, metal, stone, plastic, rubber, silicon or porcelain.

4. A support as claimed in any of claims 1 to 3, wherein a transparent support material selected from the group of glass, quartz, silicon or plastic is used.

5. An analytical measurement method which comprises applying liquid analysis samples in the hydrophilic measurement zones of a support as claimed in any of claims 1 to 4, overlaying the hydrophilic measurement zones with a hydrophobic liquid and performing the analysis.

6. An analytical measurement method as claimed in claim 5, wherein the analytical measurement is carried out in an atmosphere which is virtually saturated with water vapor.

7. An analytical measurement method as claimed in claim 5 or 6, wherein the analytical measurement is carried out while cooling the support.

8. The use of a support as claimed in any of claims 1 to 4 in diagnostic methods, in research looking for active substances, in combinatorial chemistry, in crop protection, in toxicology or in environmental protection.

**Revendications**

1. Support solide pour méthodes de mesure analytiques, constitué essentiellement d'une matière de support solide inerte sur laquelle des régions de mesure hydrophiles, portant éventuellement une charge superficielle, sont séparées les uns des autres par au moins un revêtement hydrophobe discontinu, 10 points de mesure par $cm^2$ ou plus étant disposés sur le support.

2. Support solide selon la revendication 1, **caractérisé par le fait que** les régions de mesure hydrophiles appliquées sur le support sont séparées les unes des autres par des régions hydrophobes discontinues en forme d'anneaux.

3. Support selon la revendication 1 ou 2, **caractérisé par le fait que** la matière de support consiste en verre, céramique, quartz, métal, pierre, résine synthétique, caoutchouc, silicium ou porcelaine.

4. Support selon les revendications 1 à 3, **caractérisé par le fait que** l'on utilise une matière de support transparente choisie parmi le verre, le quartz, le silicium ou une résine synthétique.

5. Méthode de mesure analytique, **caractérisée par le fait que** l'on place des échantillons analytiques liquides dans les régions de mesure hydrophiles d'un support selon les revendications 1 à 4, on revêt les régions de mesure hydrophiles d'un liquide hydrophobe et on procède à l'analyse.

6. Méthode de mesure analytique selon la revendication 5, **caractérisée par le fait que** l'on procède à la mesure analytique en atmosphère pratiquement saturée de vapeur d'eau.

7. Méthode de mesure analytique selon la revendication 5 ou 6, **caractérisée par le fait que** l'on procède à la mesure analytique en refroidissant le support.

8. Utilisation d'un support selon les revendications 1 à 4, pour le diagnostic, la recherche de substances actives, la chimie combinatoire, la protection des végétaux, la toxicologie ou l'écologie.

## FIG.1

## FIG.2

FIG.3

FIG.4